# EUROPEAN PATENT APPLICATION

(11) **EP 1 376 117 A1**
(43) Date of publication of application: **02.01.2004**
(21) Application number: 02713223.2
(22) Date of filing: 28.03.2002
(51) Int. Cl.: G01N 27/416, G01N 27/406

(54) **CONCENTRATION CELL TYPE HYDROGEN SENSOR AND METHOD FOR PREPARING SOLID ELECTROLYTE CAPABLE OF CONDUCTING PROTON**

(30) Priority: 03.04.2001 JP 2001104317
(71) Applicant: TYK Corporation, Tokyo 100-0005 (JP)
(72) Inventor: FUKATSU, Norihiko, Tajimi-shi, Gifu 507-0072 (JP); KURITA, Noriaki, Nagoya-shi, Aichi 462-0846 (JP); OHASHI, Teruo, Komaki-shi, Aichi 485-0021 (JP); KATAHIRA, Koji, c/o TYK Corporation, Tajimi-shi, Gifu 507-8607 (JP); IWAMOTO, Takashi, c/o TYK Corporation, Tajimi-shi, Gifu 507-8607 (JP)
(74) Representative: Leson, Thomas Johannes Alois, Dipl.-Ing.
(86) International application number: PCT/JP2002/003069
(87) International publication number: WO 2002/082068

(57) **Abstract**

The present invention provides a hydrogen sensor including a concentration cell holding a solid electrolyte having proton conductivity. The solid electrolyte is mainly formed of α alumina in which oxide of alkaline earth metal is doped. The hydrogen sensor can measure a hydrogen concentration of measuring objects. For example, it is possible to measure a hydrogen concentration of high temperature gas or molten metal such as copper alloy, cast iron, carbon steel, and alloy steel.

## Description

### TECHNICAL FIELD

The present invention relates to a hydrogen sensor holding a concentration cell using a solid electrolyte having proton conductivity, and a process for producing a solid electrolyte having proton conductivity. The present invention in detail relates to a hydrogen sensor for being used in high temperatures and a process for producing a solid electrolyte for being used in high temperatures.

### BACKGROUND ART

Conventionally, a hydrogen sensor has been known holding a concentration cell using a perovskite type electrolyte mainly made from SrCeO₃ and CaZrO₃ to have proton conductivity. However, when the hydrogen sensor using the conventional solid electrolyte is kept in a high temperature region over 800°C, especially in a high temperature region over 1200°C, the solid electrolyte exhibits oxide ionic conductivity and electronic conductivity, and thereby it can not be used as a solid electrolyte having proton conductivity. Therefore, the conventional hydrogen sensor made from SrCeO₃ and CaZrO₃ can not substantially work as a good hydrogen sensor for measuring a hydrogen concentration of measuring objects in a high temperature region.

The present invention has been developed in view of the abovementioned circumstances. It is an object of the present invention to provide a hydrogen sensor which can work in a high temperature region. It is another object of the present invention to provide a process for producing a solid electrolyte having proton conductivity in a high temperature region.

### DISCLOSURE OF THE INVENTION

The present inventors have made earnest studies with the abovementioned object on a hydrogen sensor holding a concentration cell with a solid electrolyte having proton conductivity. Then, the present inventors have known that a solid electrolyte formed of α alumina in which oxide of alkaline earth metal is doped is preferable in constituting a hydrogen sensor holding a concentration cell; so, the present inventors have developed a hydrogen sensor having a concentration cell.

Further, the present inventors have known that when α alumina including oxide of alkaline earth metal is sintered to form a sintered body, and when the sintered body is heat-treated in a temperature region for measuring a hydrogen concentration of the measuring objects in an atmosphere including hydrogen or steam, the α alumina can be used as a solid electrolyte having proton conductivity. Then, the present inventors have developed a process for producing a solid electrolyte having proton conductivity.

The reason for producing the solid electrolyte having good proton conductivity will hereinafter be guessed ― though it is not always clear ― as follows: a solution quantity is further stabilized in dissolving alkaline earth metal or oxide of alkaline earth metal in α alumina, when the sintered body formed of α alumina is heat-treated in an atmosphere including hydrogen or steam. Still, the temperature region may be 800-1700°C for measuring a hydrogen concentration of the measuring objects.

That is to say, a hydrogen sensor according to a first invention comprises a concentration cell holding a solid electrolyte having proton conductivity, the solid electrolyte is mainly formed of α alumina in which oxide of alkaline earth metal is doped.

A hydrogen sensor according to a second invention comprises a concentration cell holding a solid electrolyte having proton conductivity, and a pair of electrodes disposed at both surfaces of the solid electrolyte, wherein the solid electrolyte is formed of α alumina in which oxide of alkaline earth metal is doped.

A process for producing a solid electrolyte having proton conductivity according to a third invention comprises the steps of: (1) obtaining a sintered body by sintering α alumina including oxide of alkaline earth metal; and (2) heat-treating the sintered body in a measuring temperature region for measuring a hydrogen concentration of a measuring object in an atmosphere including hydrogen gas or steam.

The reason why the abovementioned solid electrolyte having proton conductivity is produced is that a solution quantity is stabilized in α alumina in dissolving alkaline earth metal or oxide of alkaline earth metal, when the sintered body is heat-treated in an atmosphere including hydrogen gas or steam.

A process for producing a solid electrolyte according to a fourth invention comprises the steps of: (1) obtaining a sintered body by sintering α alumina including oxide of alkaline earth metal; and (2) heat-treating the sintered body in a temperature of 800-1700°C in an atmosphere including hydrogen gas or steam. The reason why the abovementioned solid electrolyte having good proton conductivity is produced is that a solution quantity is stabilized in α alumina in dissolving alkaline earth metal or oxide of alkaline earth metal. The reason is guessed in such a way.

The hydrogen sensor according to the first and the second invention comprises a concentration cell holding a solid electrolyte mainly formed of α alumina in which the oxide of alkaline earth metal is doped. The solid electrolyte exhibits proton conductivity in a high temperature region, since hydrogen dissolves in α alumina with forming a weak "O-H" coupling. The hydrogen sensor of the present invention works for measuring a hydrogen concentration in a high temperature region by means of such proton conductivity. Therefore, the hydrogen sensor of the present invention can measure a hydrogen concentration of measuring objects, based on an electromotive force generated in a high temperature region ― generally in a range of 800-1700°C, especially in a range of 1200-1700 °C . Thus, the hydrogen sensor can measure a hydrogen concentration of the measuring objects. The measuring objects includes gas having high temperatures and metal molten such as copper molten metal and ferrous molten metal.

### .(Modes of the Present Invention)

The preferable modes of the present invention will hereinafter be explained. The solid electrolyte having proton conductivity is mainly formed of α alumina in which oxide of alkaline earth metal is doped. That is to say, the solid electrolyte of the present invention contains α alumina and oxide of alkaline earth metal―at least one selected from the group consisting of Be, Mg, Ca, Sr, Ba, and Ra. The content quantity of the oxide of alkaline earth metal can generally be set in a range of 0.01-1.0 mol %, especially in a range of 0.02-0.9 mol %, in a range of 0.03-0.8 mol %, or in a range of 0.03-0.6 mol %. However, it is not limited to these. Still, the word of "dope" can contain a doping form of common awareness, and the natural inclusion of the oxide of alkaline earth metal. One example of the process for producing a solid electrolyte having proton conductivity can include the steps of: (1) preparing a powder of α (alpha) alumina including a specified quantity of the oxide of alkaline earth metal ― it is generally in a range of 0.01-1. 0 mol %; (2) press-forming the powder of α alumina to form a green body; (3) sintering the green body to form a sintered body; and (4) heat-treating the sintered body in an atmosphere including hydrogen or steam and in a measuring temperature region for measuring a hydrogen concentration of measuring objects ― generally in a range of 800-1700 °C, especially in a range of 1200-1400 °C.

Another example of the process for producing a solid electrolyte having proton conductivity can include the steps of: (1) preparing a power of α alumina in which the oxide of chemical element is doped in a range of 0.01-1.0 mol %; (2) press-forming the powder of α alumina to form a green body; (3) sintering the green body to form a sintered body; and (4) heat-treating the sintered body in a measuring temperature region for measuring a hydrogen concentration of measuring objects and in an atmosphere including hydrogen gas or steam. Such chemical element is at least one selected from alkaline earth metal of Be, Mg, Ca, Sr, Ba, and Ra; and at least one selected from the group consisting of Cr, Fe, Co, W, Ga, In, and Ti. Still, the measuring temperature region for measuring a hydrogen concentration of measuring objects can be set in a range of 800-1700 °C, especially in a range of 800-1400°C, or in a range of 1200-1400°C. The reason why the heating temperature is set at the temperature region for measuring a hydrogen concentration of measuring objects is that the proper quantity of the doped material is dissolved in α alumina, since a solution quantity of the doped material is varied depending on temperature.

According to other example, the chemical element powder selected from the group of alkaline earth metal ― Be, Mg, Ca, Sr, Ba, and Ra ― is doped in α alumina, and thereby the α alumina is sintered in an oxidation atmosphere such as air. This case can be substantially equivalent that oxide of alkaline earth metal is doped in α alumina since alkaline earth metal is oxidized in the oxidation atmosphere.

Based on the experiment carried out by the present inventors, the reason why the solid electrolyte produced by the abovementioned process exhibits proton conductivity is that hydrogen is dissolved with forming a weak " O-H" coupling in α alumina in which the oxide of alkaline earth metal is doped.

Generally, time for the abovementioned heat treatment is dependent on a purity of α alumina. Based on the experiments carried out by the present inventors, when the purity of α alumina was 99.6% at mole ratio, the heat treatment required 21 hours. When the purity of α alumina was 99% or less at mole ratio, the heat treatment required over 100 hours. The production cost is disadvantageous when time for the heat treatment is long. The abovementioned heat treatment is effective in obtaining the hydrogen sensor having a good ability. However, when the hydrogen sensor does not request a good ability, the abovementioned heat treatment can be abolished, or can be carried out in a short time. Therefore, when the hydrogen sensor does not request a good ability, time for the heat treatment generally can be set at less than 5 hours, or less than 1 hours ― however, it is not limited to these. Still, when the doped oxide of alkaline earth metal is magnesium oxide, the hydrogen sensor exhibits high sensitivity, based on the experiments carried out by the present inventors.

The process for producing the solid electrolyte according to the present invention is not limited to the process of the third and the fourth present invention. In the preferable hydrogen sensor, an electrode can be disposed by coating paste film formed of at least one selected from the group of Ni, Pt, Au, and Pd as a major component. The electrode can be formed by a normal coating method, a PVD method, or a CVD method. The electrode can also be disposed by coating a paste including at least one of Ni, Pt, Au, Pd, etc. as a major component on the surface of the solid electrolyte, and by baking the coated paste so as to form the porous electrode in a reducibility atmosphere and in a high temperature, for example over 800 °C ―a paste coating method. This case preferably allows hydrogen to easily invade the electrode since the electrode is porous. Still, the electrode made of Pt or Au which is hardly oxidized can be baked in air.

In the case of the hydrogen sensor for measuring hydrogen quantity included in molten metal, the molten metal itself in which the hydrogen sensor is soaked becomes a measuring pole. In this case, the electrode is sometimes not formed on the surface of the solid electrolyte being in contact with the molten metal. In this case, it is thought that protons and electrons generate in the molten metal in contact with the solid electrolyte.

In the case where hydrogen concentration is measured by using the hydrogen sensor in which a pair of electrodes are disposed on the both surfaces of the solid electrolyte, when a measuring object, namely, gas including hydrogen and having high temperatures, for instance over 1200°C, comes into contact with the electrode of the solid electrolyte, namely, the measuring electrode, formed on the one surface of the solid electrolyte; therefore, hydrogen generates protons and electrons. According to the solid electrolyte formed of α alumina in which the oxide of alkaline earth metal is doped, since hydrogen dissolves in the α alumina with forming a weak "O- H" coupling, the solid electrolyte exhibits proton conductivity even in a high temperature region over 1200°C. Therefore, protons can move through the solid electrolyte to reach the other electrode ― reference electrode ― disposed on the opposite side of the abovementioned measuring electrode.

In the meantime, since the electrons pass through a lead wire which connects the measuring electrode and the reference electrode, the electrons reach the reference electrode. The protons which reach the reference electrode generate hydrogen with electrons. By this reaction, the electrons move from the measuring electrode side to the reference electrode side. The moving energy is proportional to a logarithm of partial pressure of the hydrogen gas. So, a partial pressure of hydrogen gas, namely, a hydrogen concentration of the gas including hydrogen ― the measuring object ― is detected by measurement of an electromotive force by use of an electromotive force measuring-means ( for instance voltmeter) with the lead wire.

### BRIEF DESCRIPTION OF THE DRAWINGS

Figure 1 is a sectional view which typically shows a condition that a hydrogen sensor concerning a first embodiment is installed in a partition of a measuring gas flow path.
Figure 2 is a graph which shows a relationship between an electromotive force of the hydrogen sensor concerning the embodiment and a partial pressure of hydrogen in logarithm.
Figure 3 is a sectional view which typically shows a hydrogen sensor.
Figure 4 is a graph which shows a relationship between measuring time and an electromotive force (EMF) based on a hydrogen concentration.
Figure 5 is a graph which shows a relationship between a measuring time and an electromotive force (EMF).
Figure 6 is a graph which shows a relationship between a measuring time and a partial pressure of hydrogen.
Figure 7 is a sectional view which concerning a fourth embodiment using a consumerism type hydrogen sensor.
Figure 8 is a graph which shows IR analysis results of α alumina including magnesium oxide and strontium oxide.

### BEST MODE FOR CARRYING OUT THE INVENTION

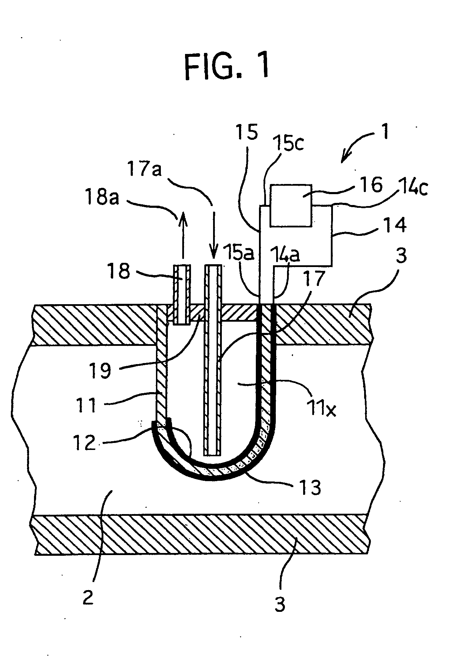

A first embodiment according to the present invention will be hereinafter described concretely. Figure 1 shows a sectional view including a hydrogen sensor installed on the partition of a measuring gas flow path having a temperature of about 1400°C. The hydrogen sensor concerning the present embodiment includes: (1) a solid electrolyte 11 forming a cavity 11x and having a cylindrical shape including a bottom; (2) a reference electrode 12 formed of a porous film and disposed on an inner surface of the solid electrolyte 11; (3) a measuring electrode 13 disposed on an external surface of the solid electrolyte 11; (4) a means 16 for measuring an electromotive force and having one end connected with the reference electrode 12 with a lead wire 15 and the other end connected with a measuring electrode 13 with a lead wire 14; (5) a lid member 19 for stabling a partial pressure of hydrogen in the cavity 11x facing the reference electrode 12; and (6) an inlet pipe 17 and an outlet pipe 18 being fixed on the lid member 19. Since the inlet pipe 17 and the outlet pipe 18 are installed on the hydrogen sensor, they can work as a means for supplying a standard material to the reference electrode 12 of the solid electrolyte 11. The solid electrolyte 11 is formed by way of the steps of: sintering α alumina including the oxide of alkaline earth metal to obtain a sintered body; and heat-treating the sintered body in an atmosphere including hydrogen gas or steam at a temperature region of 800-1700°C. The solid electrolyte 11 exemplifies 0.3mm-3mm, especially 0.5mm-2mm, in thickness. The thickness is not limited to these.

The tip of the solid electrolyte 11 constituting the hydrogen sensor 1 is inserted into the measuring gas flow path 2 along which the gas flows for working as a measuring object. The upper part of the hydrogen sensor 1 is fixed on the partition 3 (a sensor-attaching portion) forming the measuring gas flow path 2 by a mounting (not showing). The measuring electrode 13 is exposed in the gas having a temperature of about 1400°C and flowing along the measuring gas flow path 2. The predetermined quantity of the reference gas 17a is supplied into the cavity 11x from the upper end of the inlet pipe 17 by use of a mass flow controller and a steam saturation equipment (not shown). The reference gas 17a is exhausted from the outlet pipe 18 as exhaust gas 18a. The reference gas 17a, including H₂ with a fixed partial pressure of hydrogen and steam (H₂O), can work as a standard material of the concentration cell.

The solid electrolyte 11 having a protecting tube shape is produced by heat-treating an alumina tube including magnesium oxide (MgO) of about 0.15 mol % as an impurity. This alumina tube was heat-treated at a temperature of 1400 °C for 21 hours in an atmosphere including 2% H₂O and 1% H₂ volume ratio. The solid electrolyte 11 is formed of this heat-treated alumina tube.

Then, after a platinum paste is coated on the inner surface and the external surface of the solid electrolyte 11, the platinum paste is baked at a temperature of 1500°C in an oxidation atmosphere so as to form the reference electrode 12 and the measuring electrode 13. The reference electrode 12 and the measuring electrode 13 are porous. The lead wires 14, 15, working as a conductive path, are formed of platinum wires. One end part 14a of the lead wire 14 is connected with the measuring electrode 13 by use of solder paste. One end part 15a of the lead wire 15 is connected with the reference electrode 12. Other end parts 14c, 15c of the lead wires 14, 15 are connected with the voltmeter which works as the means 16 for measuring an electromotive force. The inlet pipe 17, the outlet pipe 18, and the lid member 19 are formed of sintered α alumina having a purity of 95% at mole ratio.

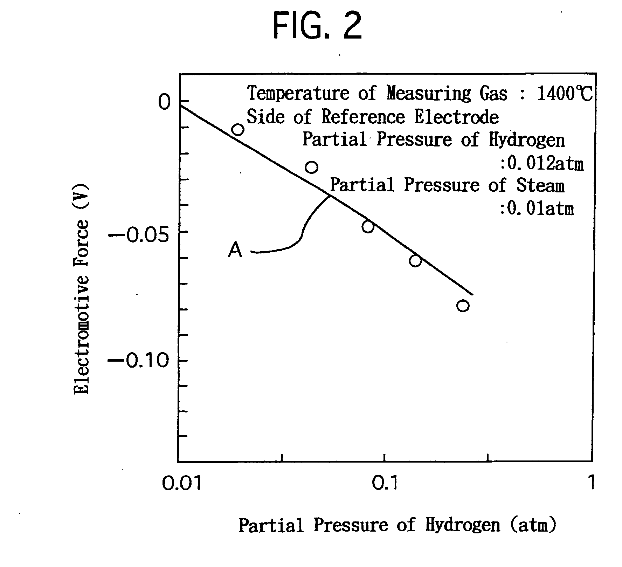

Figure 2 shows a graph for showing a relationship between an electromotive force measured by the hydrogen sensor 1 and a partial pressure of hydrogen of the gas of the measuring gas flow path 2. The partial pressure of hydrogen was set at 0.012 atm, and the partial pressure of steam was set at 0.01 atm in the reference electrode 12. The characteristic line A of Figure 2 shows the relationship between the electromotive force measured by the hydrogen sensor 1 and the partial pressure of hydrogen. In this case, the gas flowing along the measuring gas flow path 2 is varied in the partial pressure of hydrogen. The characteristic line A of Figure 2 shows a linear relation between the electromotive force and the partial pressure of hydrogen. As shown at the characteristic line A of Figure 2, it is understood that the hydrogen sensor can work even at a high temperature of 1400°C, when the gas has a partial pressure of hydrogen of 0.01 atm or more.

### (Comparable Example)

In a comparable example, a solid electrolyte was formed of an alumina tube being sold in market without the heat treatment concerning the present embodiment. The hydrogen sensor of the comparable example was made by this alumina tube. Using the hydrogen sensor concerning the comparable example, the relationship was examined between the partial pressure of hydrogen and the electromotive force. This comparable example did not work as a good hydrogen sensor, because the measured electromotive force was unstable to be greatly varied depending on time.

### (Second Embodiment)

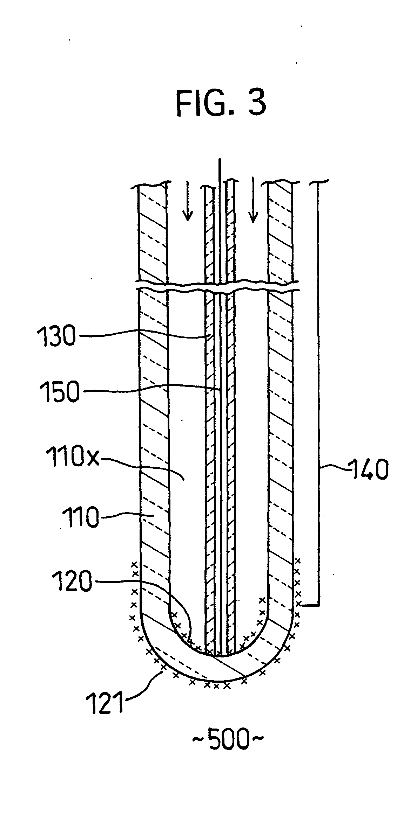

Figure 3 shows a second embodiment. The hydrogen sensor having a concentration cell concerning the second embodiment includes: (1) a solid electrolyte 110 having a cylindrical shape including a bottom (thickness of the bottom wall: 0.8 mm) and having proton conductivity; (2) an electrode 120 formed by coating a platinum paste on an inner surface of the bottom portion of cavity 110x of the solid electrolyte 110; (3) an electrode 121 formed by coating a platinum paste on an external surface of the bottom portion of the solid electrolyte 110, (4) an inserting tube 130 disposed in the cavity 110x and having a lead wire 150 connected with the electrode 120; and (5) a lead wire 140 connected with the electrode 121. The solid electrolyte 110 is mainly formed of the sintered body made of α alumina in which the oxide ― magnesium oxide ― of alkaline earth metal is included in a range of 0.05-0.20 mol % .

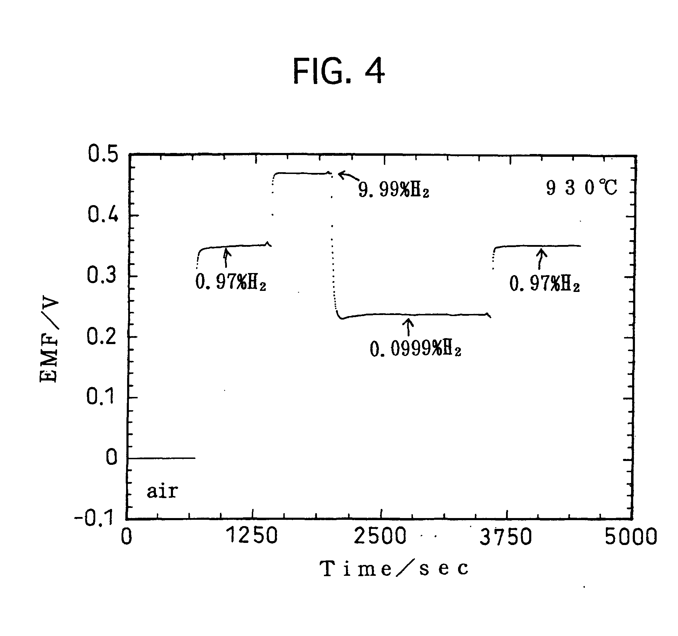

The solid electrolyte 110 is formed by way of the steps of: heating α alumina including the oxide of alkaline earth metal to form a sintered body; and heat-treating the sintered body in an atmosphere including hydrogen or steam at a temperature of 800-1700°C. Air is inserted to the cavity 110x of the solid electrolyte 110. Since the air has a low partial pressure of hydrogen, the air can work as a reference gas ― standard material ― in measuring a hydrogen concentration of gas 500 including hydrogen, a measuring object, by use of the hydrogen sensor. Then, this hydrogen sensor can measure a hydrogen concentration in the gas 500 including hydrogen―argon gas including hydrogen having a temperature of 930 °C―which works as a measuring object. In this case, the electromotive force was measured by use of the voltmeter connected with the lead wires 140, 150. The measured result is shown in Figure 4. In Figure 4, the horizontal axis shows a time (unit: second) and the vertical axis shows an electromotive force (unit: V) .

As shown in Figure 4, the electromotive force stably showed 0.34-0.36 V, when the gas was including 0.97% H₂ at volume ratio. The electromotive force stably showed about 0. 47V, when the gas was including 9.99% H₂ at volume ratio. The electromotive force stably showed about 0.24V, when the gas was including 0.0999% H₂ at volume ratio. As shown in Figure 4, when the air in the cavity 110x is utilized as the standard material, the electromotive force is generated in proportion to the hydrogen concentration included in the gas 500 which works as the measuring object. Thus, this hydrogen sensor is to be effectively utilized.

### (Third Embodiment)

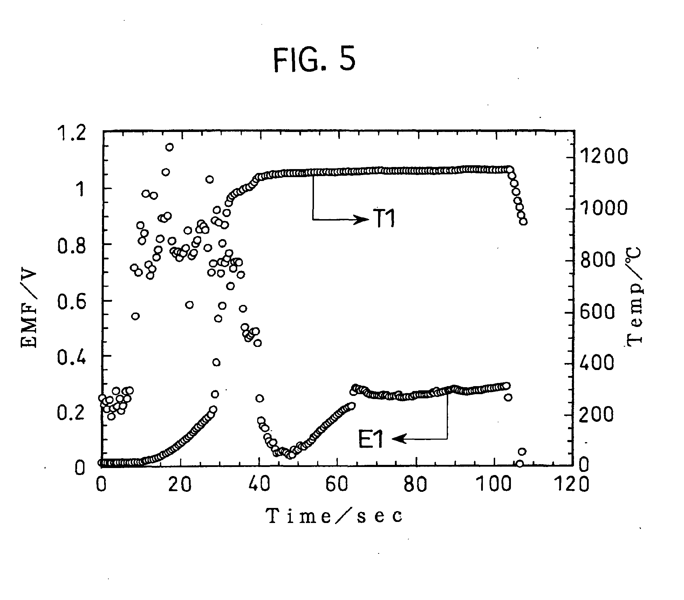

A third embodiment uses a hydrogen sensor (shown in Figure 3) having a concentration cell being similar to the second embodiment. In the third embodiment, the standard material is inserted in the cavity 110x of the solid electrolyte 110 having a cylindrical shape and having proton conductivity. The standard material was the mixture of the oxide including lantern, strontium, and cobalt with aluminum phosphate. The mixing ratio of the mixture is as follows: aluminum phosphate : oxide including lantern, strontium, and cobalt =1 : 9, weight ratio. Then, the hydrogen sensor having the concentration cell measured a hydrogen concentration as a electromotive force in the measuring object, namely the copper alloy molten metal having a temperature of 1180 °C . Figure 5 shows the results. In Figure 5, the horizontal axis shows a time (unit: second) and the vertical axis shows an electromotive force (V).

The characteristic line T1 of Figure 5 shows a temperature of the measuring object. The characteristic line E1 shows an electromotive force. As shown in Figure 5, after about 40 seconds from the starting of measurement began to stabilize the electromotive force. After about 65 seconds from the starting of measurement stabilized the electromotive force; so, the hydrogen sensor can measure a hydrogen concentration being included in the molten metal.

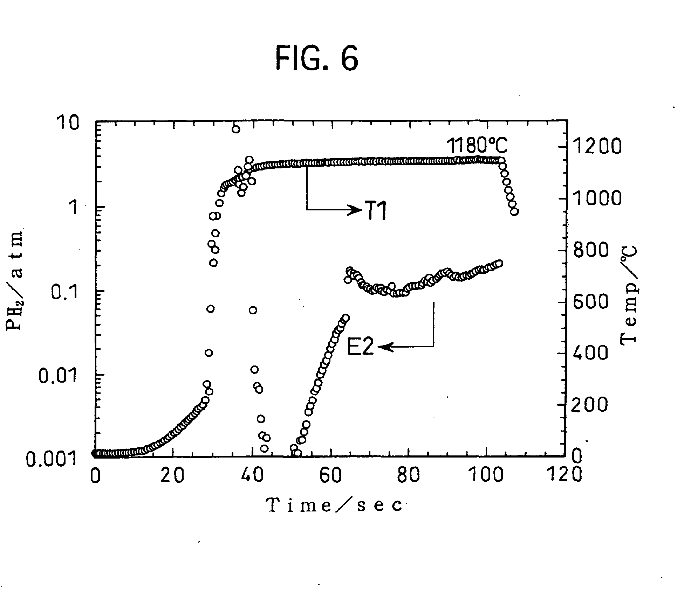

Figure 6 shows the results of the partial pressure of hydrogen of molten metal ― a measuring object ― based on the results of Figure 5. In Figure 6, the characteristic line T1 shows a temperature of the measuring object. The characteristic line E2 shows a partial pressure (unit : atm) of hydrogen based on the electromotive force. As shown at the characteristic line E2 of Figure 6, the partial pressure of hydrogen of the measuring object was near 0.1-0.27 atm. The hydrogen concentration of the same measuring object was similarly measured by use of a hydrogen sensor having a solid electrolyte formed of a conventional Ca-Zr-In-O system, namely CaZr_{0.9}In_{0.1}O₃₋ₓ, as another comparable example. In another comparable example, the hydrogen concentration was 0.215 atm. That is to say, the measured results were correspondent between the hydrogen sensor concerning the another comparable example and the hydrogen sensor concerning the third embodiment. So, the hydrogen concentration can be measured in a high temperature region by use of the hydrogen sensor concerning the third embodiment with the solid electrolyte 110 produced by the sintered body mainly formed of α alumina including the oxide of alkaline earth metal-magnesium oxide.

### (Fourth Embodiment)

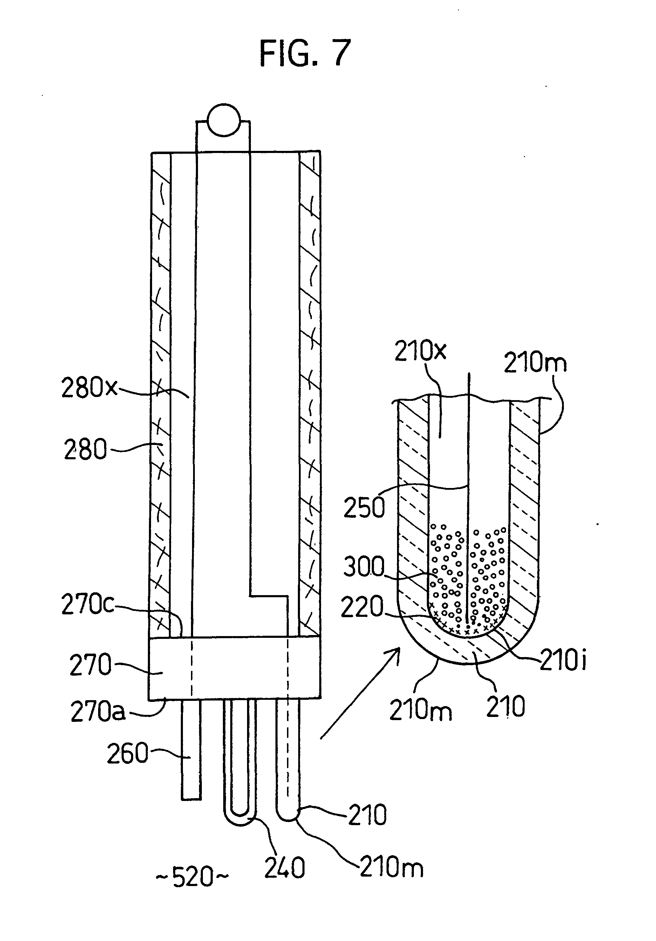

Figure 7 shows a fourth embodiment using a consumerism type hydrogen sensor. As shown in Figure 7, the hydrogen sensor concerning the fourth embodiment having a concentration cell includes: (1) a solid electrolyte 210 having proton conductivity and having a cylindrical shape concluding a bottom wall (thickness of the bottom wall: 0.8 mm); (2) an electrode 220 formed of Pt system on an inner surface 210i of the bottom of the cavity 210x of the solid electrolyte 210; (3) an inserting tube 230 made of refractory material with the lead wire 250 connected with the electrode 220; (4) an external electrode 260; and (5) a holding portion 270 formed of refractory material.

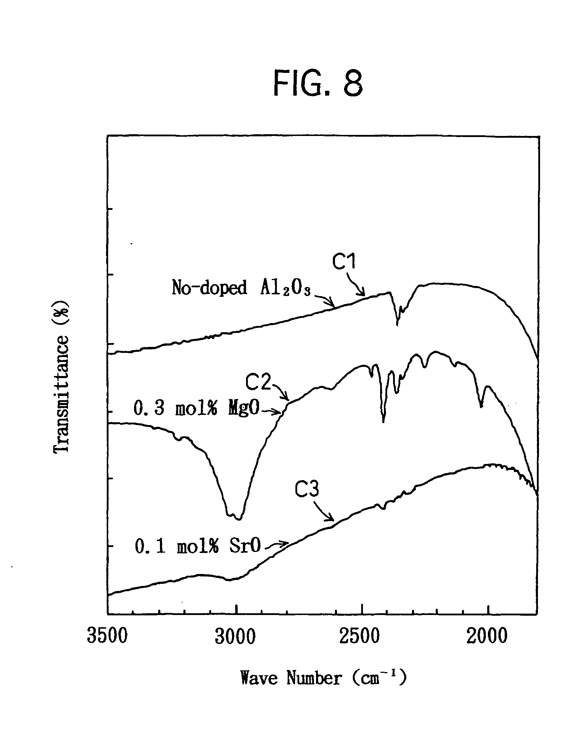

The solid electrolyte 210, thermocouples 240 for thermometry, and the external electrode 260 (electrode) are installed at one surface 270a of the holding portion 270. The sleeve-shaped barrel 280 having the cavity 280x is held on the other surface 270c of the holding portion 270. The solid electrolyte 210 is produced by the process including the steps of: sintering α alumina containing the oxide of alkaline earth metal, magnesium oxide, in a range of 0.01-1.0 mol % so as to obtain a sintered body; and heat-treating the sintered body in an atmosphere having hydrogen or steam in a range of 800-1700°C. The barrel 280 can be formed of paper which is low cost and works as a burning material. Or, the barrel 280 can be formed of inorganic material such as alumina fiber. The powder of standard material 300 is inserted in the bottom of the cavity 210x of the solid electrolyte 210 having a cylindrical shape. The standard material 300 can exemplify the mixture of oxide containing lantern, strontium, and cobalt with aluminum phosphate ― La_{0.4} Sr_{0.6}CoO₃ and AlPO₄•xH₂O. The hydrogen sensor can measure a hydrogen concentration of the measuring object 520 by the electromotive force based on the hydrogen concentration of the standard material 300 and the hydrogen concentration of the measuring object 500 being in contact with the external surface 210m of the solid electrolyte 210. The present inventors formed α alumina including magnesium oxide (0.3 mol% MgO) based on the embodiment, and then, they analyzed the α alumina including magnesium oxide with an IR-analyzing apparatus. Similarly, the present inventors formed α alumina including strontium oxide (0.1 mol% SrO). The present inventors analyzed the α alumina including strontium oxide (0.1 mol% SrO) and no-doped α alumina with the IR-analyzing apparatus. Figure 8 shows the results. In Figure 8, the vertical axis shows a transmittance (%), and the horizontal axis shows a wave number ₀ The word of "transmittance" means a permeability of an infrared ray. The word of "wave number" means a wave number of an infrared ray.

In Figure 8, the characteristic line C1 shows the result of no-doped α alumina. The characteristic line C2 shows the result of the α alumina in which magnesium oxide is doped ― 0.3mol% MgO. The characteristic line C3 shows the result of the α alumina in which strontium oxide is doped ― 0.1 mol% SrO. The characteristic line C2 greatly falls when the wave number is near 3000 in Figure 8. This fall means that proton conductivity is generated. Since the wave number falls near 3000 based on the characteristic line C3, proton conductivity is generated in the α alumina in which strontium oxide is doped ― 0.1 mol% SrO.

In addition, the present invention is not limited only to the abovementioned embodiments shown in the drawings. The quantity of the oxide of alkaline earth metal can be set not only in a range of 0.01-1.0 mol % but also in a range of 0.01-2.0 mol %. The hydrogen concentration of the measuring object can be measured, even when α alumina in which not only magnesium oxide and strontium oxide but also oxide of other alkaline earth metal is doped. The solid electrolyte with proton conductivity can have not only a cylindrical shape but also a plate shape. In addition, a measuring temperature region of the hydrogen concentration by using the hydrogen sensor can generally be in a range of 800-1700 °C. However, a measuring temperature region is not limited to this range ―it allows about 600°C -800°C. The gas for the heat-treatment is the atmosphere including 2% H₂O and 1% H₂ volume ratio in the abovementioned embodiment ―the gas for the heat-treatment is not limited to this. Thus, the steam gas may be supplied into a furnace for heat-treating α alumina as the gas for the heat-treatment. Also, the hydrogen gas may be supplied into the furnace for heat-treatment.

The gas for the heat-treatment may include 0.01% H₂O, or 0.01% H₂ at volume ratio ― an upper limit may be 100% H₂. The gas for the heat-treatment may be a saturated vapor (100% H₂O). Generally, it is possible to use a mixed atmosphere of 0. 5-10% H ₂O and 0.5-10% H₂ as gas for the heat-treatment. The present invention is applied to a consumerism type hydrogen sensor or a permanent type hydrogen sensor.

### INDUSTRIAL UTILITY FIELD

The present invention can constitute hydrogen sensors for measuring a hydrogen concentration of a mesuring object which exemplifies gas or molten metal including copper alloy, cast iron, carbon steel, alloy steel, etc.

## Claims

1. A hydrogen sensor comprising a concentration cell holding a solid electrolyte having proton conductivity,
wherein said solid electrolyte is mainly formed of α (alpha) alumina in which oxide of alkaline earth metal is doped.

2. A hydrogen sensor comprising a concentration cell holding a solid electrolyte having proton conductivity, and
a pair of electrodes disposed at both surfaces of said solid electrolyte,
wherein said solid electrolyte is mainly formed of α (alpha) alumina in which oxide of alkaline earth metal is doped.

3. The hydrogen, sensor according to claim 1 or 2, wherein said α (alpha) alumina includes said oxide of alkaline earth metal in a range of 0.01-1.0 mol%.

4. A process for producing a solid electrolyte, comprising the steps of:
obtaining a sintered body by sintering α (alpha) alumina including oxide of alkaline earth metal; and
heat-treating said sintered body in a measuring temperature region for measuring a hydrogen concentration of a measuring object in an atmosphere including hydrogen gas or steam.

5. The process for producing a solid electrolyte according to claim 4, wherein said oxide of alkaline earth metal is magnesium oxide.

6. The process for producing a solid electrolyte according to claim 4, wherein said α (alpha) alumina includes said oxide of alkaline earth metal in a range of 0.01-1.0 mol%.

7. A process for producing a solid electrolyte having proton conductivity, comprising the steps of:
obtaining a sintered body by sintering α (alpha) alumina including oxide of alkaline earth metal; and
heat-treating said sintered body in a temperature of 800-1700 °C in an atmosphere including hydrogen gas or steam.

8. The process for producing a solid electrolyte according to claim 7, wherein said oxide of alkaline earth metal is magnesium oxide.

9. The process for producing a solid electrolyte according to claim 7, wherein said α (alpha) alumina includes said oxide of alkaline earth metal in a range of 0. 01-1.0 mol%.
